# EUROPEAN PATENT APPLICATION

(11) **EP 4 186 456 A1**
(43) Date of publication of application: **31.05.2023**
(21) Application number: 21210817.9
(22) Date of filing: 26.11.2021
(51) Int. Cl.: A61B 34/20, A61B 17/00

(54) **MULTI-DIMENSIONAL TOOL ADJUSTMENT BASED ON ACOUSTIC SIGNAL**

(71) Applicant: Technische Universität München, 80333 München (DE)
(72) Inventor: Navab, Nassir, 81247 Munich (DE); Matinfar, Sasan, 80689 Munich (DE); Salehi, Mehrdad, 80687 Munich (DE)
(74) Representative: Lucke, Andreas

(57) **Abstract**

The invention refers to a method for generating an acoustic calibration signal for adjusting a tool in a plurality of dimensions relying on acoustic feedback. Each dimension of the plurality of dimensions corresponds to a respective degree of freedom of the tool. The method comprises generating the calibration acoustic signal having an acoustic property for each dimension of the plurality of dimensions, wherein each of the acoustic properties varies towards a corresponding predetermined value when the tool is being adjusted in the corresponding dimension towards a corresponding predetermined target adjustment. The invention further refers to a related method and to a related alignment system for adjusting a tool in a plurality of dimensions.

## Description

### FIELD OF THE INVENTION

The present invention is in the field of tool adjustment. In particular, the invention refers to a method for generating an acoustic calibration signal for adjusting a tool in a plurality of dimensions, to a related method for adjusting a tool in a plurality of dimensions, and to a related alignment system for adjusting a tool in a plurality of dimensions.

### BACKGROUND OF THE INVENTION

In typical tool adjustment tasks, an operator of a tool, for example a medical or industrial tool, needs to position and/or orient the tool or a part thereof according to a respective target position and/or orientation, for example with respect to a given reference frame. The reference frame may for example be given by another tool, by a workpiece or by the body of a patient. In order to bring the tool from a non-adjusted configuration to an adjusted configuration, it is typically necessary for an operator of the tool to rely on real-time visual or haptic perception, possibly mediated by corresponding assistance systems. This however implies that the visual or haptic perceptual capacities of the operator remain occupied during the adjustment of the tool, for which they cannot be used for other tasks during the adjustment of the tool.

Thus, there is room for technical improvement in the field of tool adjustment.

### SUMMARY OF THE INVENTION

The present invention refers to a technique for tool adjustment based on using an acoustic calibration signal as an adjustment guide designed for allowing an operator of the tool to adjust the tool relying on auditory perceptual information, i.e. on perceptual information perceived via their sense of hearing. This is achieved by a method for generating an acoustic calibration signal for adjusting a tool in a plurality of dimensions according to claim 1, by a method for adjusting a tool in a plurality of dimensions according to claim 12 and by an alignment system for adjusting a tool in a plurality of dimensions according to claim 13.

A first aspect of the invention refers to a method for generating an acoustic calibration signal for adjusting a tool in a plurality of dimensions according to claim 1. The method may be a computer-implemented method. The acoustic calibration signal may be or correspond to an audible signal, i.e. to an acoustic signal perceivable by a person. However, the acoustic calibration signal may also be a correspond to an electronic audio signal encoding a corresponding audible signal, i.e. an electronic signal configured for being provided to some piece of electronic equipment, in particular an amplifier and/or a loudspeaker, and for triggering the generation of a corresponding audible signal. The previously mentioned electronic audio signal may be an analogue signal or a digital signal.

Each dimension of the aforementioned plurality of dimensions corresponds to a respective degree of freedom of the tool. For example, for a tool that is movable in 2d-space, the plurality of dimensions may correspond to a position of the tool in a 2d-plane, such that a first dimension of the plurality of dimensions may correspond to a first positional dimension and a second dimension of the plurality of dimensions may correspond to a second positional dimension. In other words, the first and second dimensions may for example correspond to a pair of coordinates for describing the position of the tool on a plane, for instance to a pair of XY Cartesian coordinates or to a pair of polar coordinates. If the tool is further rotatable in said 2d-space around a given axis, a third dimension of the plurality of dimensions may correspond to a rotation angle of the tool around said given axis.

As a further example, if a tool is freely movable and orientable in 3d-space, the plurality of dimensions may comprise six dimensions: three dimensions corresponding to a position of the tool in 3d-space, for example corresponding to a triple XYZ of spatial coordinates such as Cartesian, cylindrical or spherical coordinates, and three dimensions corresponding to an orientation of the tool in space, for example corresponding to three angles respectively corresponding to pitch, yaw and roll.

Notably a pitch angle, as used commonly and in the present application to designate a rotation angle of an object about an axis should not be confused with pitch or frequency pitch, as used commonly and in the present application to designate a perceptual property that allows the human ear to perceive a sound in a frequency-related scale as being "high" or "low".

The method according to the first aspect of the invention comprises generating an acoustic calibration signal with a plurality of acoustic properties. The plurality of acoustic properties comprises an acoustic property for each dimension of the plurality of dimensions, which may but need not necessarily be different from each other. For example, if the tool is to be aligned with respect to two degrees of freedom thereof, i.e. with respect to two dimensions, an acoustic calibration signal may be generated having at least a first acoustic property associated to the first dimension and a second acoustic property associated to the second dimension.

The acoustic properties of the plurality of acoustic properties may be simultaneously perceivable by the human sense of hearing. For example, a first acoustic property of the acoustic calibration signal may correspond to a pitch of the acoustic calibration signal and a second acoustic property of the acoustic calibration signal may correspond to a pulsing frequency or to a duty cycle of the acoustic calibration signal. "Pulsing frequency" may refer herein to an intermittency frequency of the acoustic calibration signal. The acoustic calibration signal can be perceived by the human ear as a sequence of sounds regularly repeating with periods corresponding to (the inverse of) the pulsing frequency. For example, a pulsing frequency of 1 s⁻¹ may refer to the fact that the acoustic calibration signal may be an intermittent signal alternating active periods of non-zero amplitude with inactive periods of zero amplitude periodically repeating in intervals of is. The alternating active periods of non-zero amplitude and the inactive periods of zero amplitude may have the same duration (for example 0,5 s and 0,5 s, respectively, for a pulsing frequency of 1 s⁻¹) or different durations (for example 0,8 s and 0,2 s, respectively, for a pulsing frequency of 1 s⁻¹). "Duty cycle" may refer herein to a fraction of one period of an intermittent (pulsing) acoustic calibration signal over which the signal is active, i.e. has a non-zero amplitude. A duty cycle of 0,5 corresponds to a pulsing signal in which the alternating active periods of non-zero amplitude have the same duration as the inactive periods of zero amplitude (for example 0,5 s and 0,5 s, respectively, for a pulsing frequency of 1 s⁻¹). A duty cycle of 1 corresponds to a continuous non-intermittent signal. Notably, the pitch, the pulsing frequency and/or the duty cycle of an auditory signal can be independently and/or simultaneously perceived by the human ear. For a given auditory signal, the human ear can for example identify when a pulsing frequency or a duty cycle of the auditory signal is varying (i.e. is pulsing slower or quicker) and can further independently identify, possibly at the same time, whether a pitch of the signal is varying (i.e. is becoming higher or lower).

According to the method of the first aspect of the invention, each acoustic property of the plurality of acoustic properties varies towards a corresponding predetermined value when the tool is being adjusted in the corresponding dimension towards a corresponding predetermined target adjustment. This may imply that, as an adjustment of the tool in a given dimension gets the tool closer to the respective predetermined target adjustment, the acoustic property corresponding to said given dimension converges to the corresponding predetermined value, in particular continuously and/or monotonously.

For example, the tool may be adjusted in two dimensions corresponding to two translational degrees of freedom of the tool, such that adjusting the tool in said two dimensions may correspond to setting a position of the tool in a corresponding two-dimensional plane, i.e. in a first spatial dimension (an X-direction) and in a second spatial dimension (a Y-direction).

Following with the previously mentioned example, the acoustic calibration signal may be an intermittent signal, a first acoustic property of the acoustic calibration signal associated to the first spatial dimension may correspond to a pulsing frequency of the acoustic calibration signal, and a second acoustic property of the acoustic calibration signal associated to the second spatial dimension may correspond to a pitch of the acoustic calibration signal. A predetermined target adjustment of the tool in each of the first and second spatial dimensions being adjusted may hence correspond to a respective predetermined target position in the corresponding direction, e.g. a corresponding target X-coordinate and a corresponding target Y-coordinate.

Accordingly, when the tool is moved in the first direction towards the predetermined target position in the first direction, e.g. to the corresponding target X-coordinate, the pulsing frequency of the acoustic calibration signal may for example continuously increase, such that the acoustic calibration signal converges towards a more rapidly alternating intermittent signal. Meanwhile, when the tool is moved in the second direction towards the predetermined target position in the second direction, the pitch of the acoustic calibration signal may for example continuously increase, such that the acoustic calibration signal has an increasingly higher pitch as the tool approaches the predetermined target position in the second direction. Therefore, a user of the tool receives acoustic information, in particular simultaneously, as to whether the tool is being correctly adjusted in each of the first and second dimensions, exclusively based on acoustic information, such that other perception channels of the user, in particular the visual and haptic channels, may remain free to be used for other purposes.

In some preferred embodiments, each acoustic property of the plurality of acoustic properties may vary away from the corresponding predetermined value when the tool is being adjusted in the corresponding dimension away from the corresponding predetermined target adjustment. This may imply that, as an adjustment of the tool in a given dimension gets the tool further away from the respective predetermined target adjustment, the acoustic property corresponding to said given dimension diverges from the corresponding predetermined value, in particular continuously and/or monotonously, i.e. having an opposite evolution as in the case in which the adjustment of the tool is getting closer to the respective predetermined target adjustment. This way, a user of the tool can receive acoustic feedback, not only as a confirmation that the tool is being correctly adjusted, but also as a signal that the tool is being wrongly adjusted, i.e. that it is moving away and/or not approaching the corresponding predetermined target adjustment. This further simplifies the task of correctly adjusting the tool.

According to preferred embodiments of the invention, at least one acoustic property of the plurality of acoustic properties may correspond to one of pitch, pulsing frequency, duty cycle, loudness, and tone colour of the acoustic calibration signal. Preferably, at least another acoustic property of the plurality of acoustic properties may correspond to one of pitch, pulsing frequency, duty cycle, loudness and tone colour of the acoustic calibration signal. "Loudness" may refer herein to a subjective perception of sound pressure. "Tone colour", also named "timbre", may refer herein to an acoustic property that distinguishes different types of sound production, such as choir voices and different musical instruments for example.

In some embodiments, two or more acoustic properties of the plurality of acoustic properties may correspond to (the same) one of pitch, pulsing frequency, duty cycle, loudness, and tone colour of the acoustic calibration signal. However, in some embodiments, each acoustic property of the plurality of acoustic properties may correspond to a different one of pitch, pulsing frequency, duty cycle, loudness, and tone colour of the acoustic calibration signal. It is also possible that two or more acoustic properties of the plurality of acoustic properties may correspond to (the same) one of pitch, pulsing frequency, duty cycle, loudness, and tone colour of the acoustic calibration signal while each of the rest of acoustic properties of the plurality of acoustic properties may correspond to a different one of said properties (pitch, pulsing frequency, duty cycle, loudness, and tone colour) of the acoustic calibration signal, respectively.

In preferred embodiments of the invention, at least one dimension of the plurality of dimensions may correspond to a translational degree of freedom, a rotational degree of freedom or an orientational degree of freedom of the tool. Additionally or alternatively, at least another dimension of the plurality of dimensions may correspond to another translational degree of freedom, rotational degree of freedom or orientational degree of freedom of the tool. In some embodiments, each dimension of the plurality of dimensions may correspond to a translational degree of freedom, a rotational degree of freedom or an orientational degree of freedom of the tool, respectively.

In preferred embodiments of the invention, at least one acoustic property of the plurality of acoustic properties is kept constant, optionally at the corresponding predetermined value, while the tool remains adjusted in the corresponding dimension at the corresponding predetermined target adjustment. This means that one given acoustic property of the acoustic calibration signal used for adjusting the tool, which may have reached the corresponding predetermined value due to the tool having been adjusted to the corresponding predetermined target adjustment, may remain constant as long as the tool stays adjusted in that corresponding dimension, in particular within a predefined tolerance. If instead, the tool loses adjustment in that corresponding dimension, the aforesaid acoustic property of the acoustic calibration signal may start varying again, thereby indicating to the user that the previously reached adjustment of the tool in said corresponding dimension has been lost and needs to be readjusted.

For example if a tool is being adjusted in two spatial dimensions using pitch and duty cycle as acoustic properties respectively associated to one of the two spatial dimensions, the duty cycle may evolve towards a continuous non-pulsing signal (i.e. to a duty cycle value 1) as the tool improves its adjustment with respect to a corresponding predetermined target adjustment in the second spatial dimension and may remain a continuous non-pulsing signal as long as the tool stays adjusted to said predetermined target adjustment in the second spatial dimension, in particular while the tool continues to be adjusted with respect to the first spatial dimension. If, at some point, the tool ceases being adjusted to said predetermined target adjustment in the second spatial dimension, the pulsing frequency of the acoustic calibration signal may start varying again, i.e. the acoustic calibration signal may turn into a pulsing signal again, thereby indicating to the user that the tool needs to be readjusted with respect to the second spatial dimension as well.

According to preferred embodiments, the method may further comprise, for at least one dimension of the plurality of dimensions, possibly for some or each dimension of the plurality of dimensions, generating an acoustic marker when the tool reaches the corresponding predetermined target adjustment in said at least one dimension, in particular within a predefined tolerance. An acoustic marker may be or comprise any kind of information or feature perceivable by the human ear, which may be used as a dedicated indicator to a user performing the method of the invention that the tool has been correctly adjusted in one given dimension with respect to a corresponding predetermined target adjustment.

In some embodiments, the at least one generated acoustic marker may correspond to a discontinuous variation of the corresponding acoustic property, in particular by 5% or more, preferably by 10% or more, more preferably by 20% or more or by 50% or more. Thus, the acoustic marker may be in the form of a clearly perceivable discontinuity in one of the acoustic properties of the acoustic calibration signal. For example, if a tool is being adjusted in two spatial dimensions using pitch and pulsing frequency as acoustic properties respectively associated to one of the two spatial dimensions, a sudden increase in the pitch of the acoustic signal by 50% may be used as an acoustic marker generated when the tool reaches the corresponding predetermined target adjustment in one of the spatial dimensions, whereas a sudden increase by 100% of the pulsing frequency of the acoustic signal may be used as an acoustic marker generated when the tool reaches the corresponding predetermined target adjustment in the other one of the spatial dimensions.

According to some embodiments, generating the at least one acoustic marker may comprise generating a corresponding additional acoustic signal different from the acoustic calibration signal. For example, for a dimension of the plurality of dimensions corresponding to a rotational degree of freedom of the tool, the corresponding predetermined target adjustment may correspond to a predetermined rotation angle and an acoustic marker may be generated, for example in the form of a short continuous sound with high pitch - unrelated to the acoustic calibration signal - or in the form of a short arpeggio, when the tool is rotated such that it is adjusted to the predetermined rotation angle for indicating that the tool has been correctly adjusted in said dimension.

According to preferred embodiments of the invention, at least one acoustic property of the plurality of acoustic properties, possibly more than one or each acoustic property of the plurality of acoustic properties, may vary within a corresponding predetermined range towards one end value of said corresponding predetermined range when the tool is being adjusted in the respective dimension towards the corresponding predetermined target adjustment. Preferably, said at least one acoustic property of the plurality of acoustic properties may vary within the corresponding predetermined range towards another end value (i.e. the opposite end value) of said corresponding predetermined range when the tool is being adjusted in the respective dimension away from the corresponding predetermined target adjustment.

For example, if a tool is being adjusted in two dimensions corresponding to one translational degree of freedom and one rotational degree of freedom using a pitch and a pulsing frequency of the acoustic calibration signal as acoustic properties respectively associated to the two dimensions in which the tool is to be adjusted, the pitch of the acoustic calibration signal may vary within a predetermined pitch frequency range, for example between 330 Hz and 440 Hz, while the pulsing frequency of the acoustic calibration signal may vary within a predetermined pulsing frequency range, for example between 1/1.5 Hz and 1/0.05 Hz. The pitch frequency of the acoustic calibration signal may evolve towards 440 Hz when the tool is being adjusted such that it approaches a corresponding predetermined target adjustment with respect to the translational degree of freedom. Further, the pitch frequency of the acoustic calibration signal may evolve towards 330 Hz (i.e. away from 440 Hz) when the tool is being adjusted away from the aforesaid corresponding predetermined target adjustment with respect to the translational degree of freedom. Meanwhile, the period of the pulsing acoustic calibration signal (i.e. the inverse of the pulsing frequency) may evolve towards 0.05 s when the tool is being adjusted such that it approaches a corresponding predetermined target adjustment with respect to the rotational degree of freedom and may evolve towards 1.5 s (i.e. away from 0.05 s) when the tool is being adjusted away from the aforesaid corresponding predetermined target adjustment with respect to the rotational degree of freedom.

In some embodiments, generating the at least one acoustic marker described above may comprise replacing the corresponding predetermined range by a new corresponding predetermined range different from said corresponding predetermined range. This may allow using one same acoustic property of the plurality of acoustic properties for adjusting the tool in more than one different dimensions of the plurality of dimensions.

For example, if the tool is being adjusted in two dimensions corresponding to respective translational degrees of freedom of the tool, the acoustic calibration signal may be generated having a pitch that varies within a first predetermined range, for example between 330 Hz and 440 Hz, towards a first predetermined value, for example 440 Hz, when the tool is being adjusted in one of the two dimensions towards a corresponding predetermined target adjustment. When the tool is correctly adjusted in said one of the two dimensions and reaches the corresponding predetermined target adjustment, an acoustic marker may be generated by generating the acoustic signal now having a pitch varying within a second predetermined range, for example between 550 Hz and 660 Hz, towards a second predetermined value, for example 660 Hz, when the tool is being adjusted in the other one of the two dimensions towards a corresponding predetermined target adjustment. The sudden change from the first range between 330 Hz and 440 Hz to the second range between 550 Hz and 660 Hz signals to a user of the tool performing the method of the invention that the tool is now correctly adjusted with respect to the first degree of freedom and need now to be adjusted with respect to the second degree of freedom. The pitch of the acoustic signal may continue to vary within the second predetermined range as long as the tool stays adjusted with respect to the first degree of freedom. If the tool loses adjustment with respect to the first degree of freedom, the pitch of the acoustic signal may start varying again in the first predetermined range.

According to preferred embodiments of the invention, at least a first acoustic property and a second acoustic property of the plurality of acoustic properties may correspond to pitch, the first acoustic property may vary within a first predetermined range towards an end value of the first predetermined range when the tool is being adjusted in the first dimension towards a first predetermined target adjustment, and the second acoustic property may vary within a second predetermined range, in particular different from the first predetermined range, towards one end value of the second predetermined range when the tool is being adjusted in the second dimension towards a second predetermined target adjustment. Said end value of the second predetermined range may have a predetermined multiplicity-relation with respect to said end value of the first predetermined range, such that said end value of the second predetermined range may correspond to Q times said end value of the first predetermined range, Q being a rational factor. For example, said end value of the second predetermined range may preferably be an octave, a perfect fifth or a perfect fourth of said end value of the first predetermined range. Thus, according to these preferred embodiments, a user may be able to independently adjust the tool in a first dimension associated to a first dimension of the plurality of dimensions and in the second dimension associated to a second dimension of the plurality of dimensions using only pitch as a variable acoustic property of the acoustic calibration signal. A simultaneous correct adjustment of the tool with respect to both the first and the second dimension may be perceived by the user as a consonance in the acoustic calibration signal, due to the end value of the second predetermined range, to which the second acoustic property converges as the tool approaches the second predetermined target adjustment in the second dimension, being an octave, a perfect fifth or a perfect fourth of (i.e. being harmonically consonant with) the end value of the first predetermined range, to which the first acoustic property converges as the tool approaches the first predetermined target adjustment in the first dimension. For example, the end value of the second predetermined range may be 660 Hz and the end value of the first predetermined range may be 440 Hz, 660 Hz being a perfect fifth of 440 Hz.

According to some preferred embodiments, the plurality of dimensions may comprise a first dimension corresponding to a first degree of freedom of the tool and a second dimension corresponding to a second degree of freedom of the tool, which may in particular be different from the first degree of freedom of the tool. The plurality of acoustic properties may comprise a first acoustic property and a second acoustic property different from the first acoustic property, wherein the first and second acoustic properties may in particular be simultaneously perceivable acoustic properties. For example, a first acoustic property of the acoustic calibration signal may correspond to a pitch of the acoustic calibration signal and a second acoustic property of the acoustic calibration signal may correspond to a pulsing frequency or a duty cycle of the acoustic calibration signal. In other words, the plurality of dimensions may comprise in particular two dimensions and the associated plurality of acoustic properties may correspondingly comprise in particular two different - simultaneously perceivable - acoustic properties, each associated to one of the dimensions.

According to related embodiments, the plurality of dimensions may further comprise a third dimension corresponding to a third degree of freedom of the tool. The plurality of acoustic properties may then further comprise a third acoustic property different from the first and second acoustic properties. The first, second and third acoustic properties may in particular be simultaneously perceivable acoustic properties. This may allow simultaneously adjusting the tool in three different dimensions relying only on acoustic perception. For example, the first to third dimensions may correspond to three translational degrees of freedom of the tool in three mutually orthogonal spatial directions (e.g. XYZ-coordinates). A first acoustic property of the acoustic calibration signal associated to the adjustment of the tool in the first dimension may be a pitch, a second acoustic property of the acoustic calibration signal associated to the adjustment of the tool in the second dimension may be a pulsing frequency or a duty cycle, and a third acoustic property of the acoustic calibration signal associated to the adjustment of the tool in the third dimension maybe a loudness. Notably, the human sense of hearing is able to independently perceive a pitch, a pulsing frequency and a loudness of an acoustic signal.

In some embodiments, the plurality of dimensions may further comprise a third dimension corresponding to a third degree of freedom of the tool and a fourth dimension corresponding to a fourth degree of freedom of the tool (apart from the first dimension corresponding to a first degree of freedom of the tool and the second dimension corresponding to a second degree of freedom of the tool). The first acoustic property may vary as previously described within a first predetermined range towards one end value of the first predetermined range when the tool is being adjusted in the first dimension towards a first predetermined target adjustment and preferably towards another end value of the first predetermined range when the tool is being adjusted in the first dimension away from the first predetermined target adjustment. Additionally or alternatively, the second acoustic property may vary as previously described within a second predetermined range, which may in particular be different from the first predetermined range, towards one end value of the second predetermined range when the tool is being adjusted in the second dimension towards the second predetermined target adjustment and preferably towards another end value of the second predetermined range when the tool is being adjusted in the second dimension away from the second predetermined target adjustment. Further, when the tool reaches the first predetermined target adjustment in the first dimension, in particular within a predefined tolerance, and/or reaches the second predetermined target adjustment in the second dimension, in particular within a predefined tolerance, the first acoustic property may vary within a third predetermined range different from the first predetermined range towards one end value of the third predetermined range when the tool is being adjusted in the third dimension towards a third predetermined target adjustment and preferably towards another end value of the third predetermined range when the tool is being adjusted in the third dimension away from the third predetermined target adjustment. Additionally or alternatively, the second acoustic property may vary within a fourth predetermined range different from the second predetermined range towards one end value of the fourth predetermined range when the tool is being adjusted in the fourth dimension towards the a fourth predetermined target adjustment and preferably towards another end value of the fourth predetermined range when the tool is being adjusted in the fourth dimension away from the fourth predetermined target adjustment. According to these preferred embodiments, a number of dimensions of the plurality of dimensions higher than two may be sequentially adjusted, with two or three dimensions possibly being adjusted at the same time.

As a further example, a tool may have to be adjusted in two translational dimensions, respectively corresponding to an X-direction and a Y-direction orthogonal to the X-direction, and in two rotational dimensions respectively corresponding to a pitch angle and a yaw angle of the tool. One of the translational dimensions (a first dimension) and one of the rotational dimensions (a third dimension) may both be associated with a pitch of the acoustic calibration signal (a first acoustic property) while the other one of the translational dimensions (a second dimension) and the other one of the rotational dimensions (a fourth dimension) may both be associated with a pulsing frequency or a duty cycle of the acoustic calibration signal (a second acoustic property). The acoustic calibration signal may then be used for adjusting the tool first in the two translational dimensions (i.e. in the first and second dimensions), with the pitch varying in a first predetermined pitch range towards one end value or the other depending on whether the tool is being adjusted towards or away from a corresponding target adjustment in the first translational dimension (e.g. in the X-direction) and with the pulsing frequency or the duty cycle varying in a second predetermined pulsing frequency range towards one end value or the other depending on whether the tool is being adjusted towards or away from a corresponding target adjustment in the second translational dimension (e.g. in the Y-direction). When the tool is correctly adjusted in both translational dimensions within a respective predefined tolerance, the pitch of the acoustic calibration signal may vary in a second predetermined pitch range (a third predetermined range) different from the first predetermined pitch range towards one end value or the other depending on whether the tool is being adjusted towards or away from a corresponding target adjustment in the first rotational dimension (e.g. a pitch angle) and the pulsing frequency or the duty cycle of the acoustic calibration signal may vary in a second predetermined pulsing frequency range (a fourth predetermined range) different from the first predetermined pulsing frequency range towards one end value or the other depending on whether the tool is being adjusted towards or away from a corresponding target adjustment in the second rotational dimension (e.g. a yaw angle).

According to preferred embodiments of the invention, when the tool deviates from the first predetermined target adjustment in the first dimension, in particular beyond a predefined tolerance, the first acoustic property may stop varying within the third predetermined range and retake varying within the first predetermined range towards said one end value of the first predetermined range when the tool is being adjusted in the first dimension towards the first predetermined target adjustment and preferably towards said another end value of the first predetermined range when the tool is being adjusted in the first dimension away from the first predetermined target adjustment. Additionally or alternatively, when the tool deviates from the second predetermined target adjustment in the second dimension, in particular beyond a predefined tolerance, the second acoustic property may stop varying within the fourth predetermined range and retake varying within the second predetermined range towards said one end value of the second predetermined range when the tool is being adjusted in the second dimension towards the second predetermined target adjustment and preferably towards said another end value of the second predetermined range when the tool is being adjusted in the second dimension away from the second predetermined target adjustment. Thus, the transition from the first predetermined range to the third predetermined range and/or from the second predetermined range to the fourth predetermined range may be reversible if required for readjusting the tool in a dimension in which it had previously been adjusted but in which the correct adjustment has been lost. In this case, the perceivable transition from one predetermined range to another may signal to a user that the tool needs to be readjusted with respect to one or more dimensions in which it had previously been adjusted.

It is also foreseen within the context of the present invention to extend the above concept to a higher number of dimensions, for example to a fifth and a sixth dimension. In particular, the plurality of dimensions may further comprise a fifth dimension corresponding to a fifth degree of freedom of the tool and a sixth dimension corresponding to a sixth degree of freedom of the tool (apart from the first to fourth dimensions). The first acoustic property may then vary within the first predetermined range when adjusting the tool in the first dimension and the second acoustic property may vary within the second predetermined range when adjusting the tool in the second dimension. Once the tool is adjusted in the first and/or second dimensions, the first acoustic property may then vary within the third predetermined range when adjusting the tool in the third dimension and/or the second acoustic property may vary within the fourth predetermined range when adjusting the tool in the second dimension as explained above. Additionally, when the tool reaches the first predetermined target adjustment in the first dimension and/or reaches the second predetermined target adjustment in the second dimension and/or reaches the third predetermined target adjustment in the third dimension and/or reaches the fourth predetermined target adjustment in the fourth dimension, in particular within a predefined tolerance, respectively, the first acoustic property may vary within a fifth predetermined range different from the third predetermined range and possibly also different from the first predetermined range towards one end value of the fifth predetermined range when the tool is being adjusted in the fifth dimension towards a fifth predetermined target adjustment and preferably towards another end value of the fifth predetermined range when the tool is being adjusted in the fifth dimension away from the fifth predetermined target adjustment. Additionally or alternatively, the second acoustic property may vary within a sixth predetermined range different from the fourth predetermined range, and possibly also different from the second predetermined range, towards one end value of the sixth predetermined range when the tool is being adjusted in the sixth dimension towards a sixth predetermined target adjustment and preferably towards another end value of the sixth predetermined range when the tool is being adjusted in the sixth dimension away from the sixth predetermined target adjustment. As for the first, second, third and fourth dimensions, the transition from the third predetermined range to the fifth predetermined range and/or from the fourth predetermined range to the sixth predetermined range may be reversible if required for readjusting the tool in a dimension in which it had previously been adjusted but in which the correct adjustment has been lost.

It is also foreseen within the context of the present invention to group the different dimensions of the tool in numbers different as two, for example three. For example, the first acoustic property may vary within the first predetermined range for adjusting the tool in the first dimension while the second acoustic property varies within the second predetermined range for adjusting the tool in the second dimension and while the third acoustic property varies within a third predetermined range for adjusting the tool in the third dimension. Once the tool reaches the first predetermined target adjustment in the first dimension and/or reaches the second predetermined target adjustment in the second dimension and/or reaches the third predetermined target adjustment in the third dimension, in particular within a predefined tolerance, respectively, the first acoustic property may vary within a fourth predetermined range different from the first predetermined range towards one end value of the fourth predetermined range when the tool is being adjusted in the fourth dimension towards a fourth predetermined target adjustment and preferably towards another end value of the fourth predetermined range when the tool is being adjusted in the fourth dimension away from the fourth predetermined target adjustment. Meanwhile, the second acoustic property may vary within a fifth predetermined range different from the second predetermined range towards one end value of the fifth predetermined range when the tool is being adjusted in the fifth dimension towards a fifth predetermined target adjustment and preferably towards another end value of the fifth predetermined range when the tool is being adjusted in the fifth dimension away from the fifth predetermined target adjustment. Additionally or alternatively, the third acoustic property may vary within a sixth predetermined range different from the third predetermined range towards one end value of the sixth predetermined range when the tool is being adjusted in the sixth dimension towards a sixth predetermined target adjustment and preferably towards another end value of the sixth predetermined range when the tool is being adjusted in the sixth dimension away from the sixth predetermined target adjustment. The transition between the respective predetermined ranges may be reversible if required for readjusting the tool in a dimension in which it had previously been adjusted but in which the correct adjustment has been lost.

A second aspect of the invention refers to a method for adjusting a tool, for example an industrial or medical tool, in a plurality of dimensions according to claim 12. As for the first aspect of the invention, each dimension may correspond to a degree of freedom of the tool. The method may be a navigation method, in particular a surgical navigation method. The method of the second aspect of the invention comprises generating an acoustic calibration signal according to the method of any of the embodiments of the first aspect of the invention and further comprises adjusting the tool in each dimension of the plurality of dimensions based on the generated acoustic calibration signal, such that for a plurality of acoustic properties of the generated acoustic calibration signal, each acoustic property varies towards a corresponding predetermined value, in particular until each acoustic property reaches the corresponding predetermined value. Thereby, the tool can be adjusted in the plurality of dimensions with respect to a target state defined in the plurality of dimensions relying on auditory feedback provided by the acoustic calibration signal.

A third aspect of the invention refers to a system for adjusting a tool in a plurality of dimensions according to claim 13, each dimension corresponding to a degree of freedom of the tool. The system may in particular be a system configured for implementing the method according to the second aspect of the invention. The tool may be an industrial tool or a medical/surgical tool. The system may in particular be a surgical navigation system.

The system comprises a plurality of spatial markers arrangeable on a tool for marking a state of the tool in each dimension of the plurality of dimensions and a detection system configured for registering, in particular in real-time, the state of the tool in each dimension of the plurality of dimensions marked by the spatial markers, in particular with respect to a reference frame of the detection system.

"Spatial marker" may refer herein to any structure or device suitable for being detected by the detection system allowing determining a state of the tool in the plurality of dimensions or a corresponding part thereof. The "state of the tool" in each dimension of the plurality of dimensions may refer in particular to a configuration of the tool by which the position and/or orientation of the tool can be characterised with respect to a given dimension and to a corresponding degree of freedom of the tool. For example, for a dimension corresponding to a translational degree of freedom of the tool, the state of the tool may correspond to a position of the tool in space referred to that translational degree of freedom, for example to an X- or Y-coordinate. Likewise, for a dimension corresponding to a rotational degree of freedom the tool, the state of the tool may correspond to an angular position of the tool in space referred to that rotational degree of freedom. The detection system may be based on any scanning technology, in particular on a real-time tracking technology such as optical tracking, mechanical tracking, laser tracking and/or electromagnetic tracking or the like. Thus, the spatial markers, when registered by the detection system, characterise the state of the tool in the plurality of dimensions and allow the detection system to generate and process information about said state of the tool.

The system further comprises a mapping unit for determining a target state of the tool in each dimension of the plurality of dimensions with respect to a target reference frame. The "target reference frame" may refer in particular to a reference frame that takes into account a target state of the tool to be achieved after the adjustment process, in particular with respect to an external reference object, which may for example be a further tool, the body of a patient, a surgical operation set up (e.g. an operating theater) or an industrial set up. When registering the state of the tool in the plurality of dimensions based on the spatial markers, the detection system may do so in one given reference frame, for example its own reference frame. The mapping unit is configured for "translating" the information related to said given reference frame, for example the reference frame of the detection system, into information related to another reference frame, the target reference frame, which allows correctly adjusting the tool with respect to the aforesaid external object, e.g. a further tool, the body of the patient, a surgical operation set up or an industrial setup.

For example, if the alignment system of the invention is used as a surgical navigation system for adjusting a surgical tool for performing an implantation of an implant into the body of a patient, the mapping unit may be configured for correlating the state of the tool registered by the detection system as marked by the spatial markers with a target implant site in the body of the patient with respect to which the surgical tool is to be adjusted in order to perform the implantation of said implant at the correct position and/or with the correct orientation. Said correct position and/or correct orientation of the surgical tool in space may define in this exemplary case the corresponding target state of the tool.

The alignment system further comprises an adjustment unit configured for generating an acoustic calibration signal according to the method of any of the embodiments of the first aspect of the invention described above. The acoustic calibration signal is generated with a plurality of acoustic properties, wherein each acoustic property of the plurality of acoustic properties varies towards a corresponding predetermined value when the tool is being adjusted in the corresponding dimension towards a corresponding predetermined target adjustment. Thus, by means of the adjustment unit, the alignment system according to the third aspect of the invention allows generating and obtaining an acoustic calibration signal as described above with respect to the first aspect of the invention.

The target state determined by the mapping unit corresponds to a state in which the tool is adjusted in each dimension of the plurality of dimensions to the corresponding predetermined target adjustment, in particular within a predefined tolerance. Thus, the alignment system is configured for generating the acoustic calibration signal for adjusting the tool to the target state defined by the mapping unit.

The mapping unit, the adjustment unit and at least a part of the detection system may be integrated in one common processing unit, which may be hardware-based and/or software-based.

The adjustment unit may be further configured for generating the acoustic calibration signal such that each acoustic property varies away from said corresponding predetermined value when the tool is being adjusted in said corresponding dimension away from said predetermined target adjustment.

Additionally or alternatively, the adjustment unit may be further configured for generating, for at least one dimension of the plurality of dimensions, an acoustic marker when the tool reaches the corresponding predetermined target adjustment in said at least one dimension, in particular within the predefined tolerance.

Additionally or alternatively, the adjustment unit may be configured to generate the acoustic calibration signal according to any of the embodiments of the first aspect of the invention described above.

### BRIEF DESCRIPTION OF THE DRAWINGS

Fig. 1 shows a schematic illustration of an alignment system according to the third aspect of the invention.
Fig. 2 is a schematic flow diagram of a method for adjusting a tool in a plurality of dimensions according to the second aspect of the invention.
Fig. 3 and 4 are schematic flow diagrams of a method of generating an acoustic calibration signal according to the first aspect of the invention. Fig. 4 is a continuation of Fig. 3.
Figs. 5 to 9 are schematic illustrations of the time evolution of respective acoustic properties of an acoustic calibration signal generated by a method according to embodiments of the invention.

### DETAILED DESCRIPTION OF PREFERRED EMBODIMENTS

For the purposes of promoting an understanding of the principles of the invention, reference will now be made to a preferred embodiment illustrated in the drawings, and specific language will be used to describe the same. It will nevertheless be understood that no limitation of the scope of the invention is thereby intended, such alterations and further modifications in the illustrated apparatus and such further applications of the principles of the invention as illustrated therein being contemplated as would normally occur now or in the future to one skilled in the art to which the invention relates.

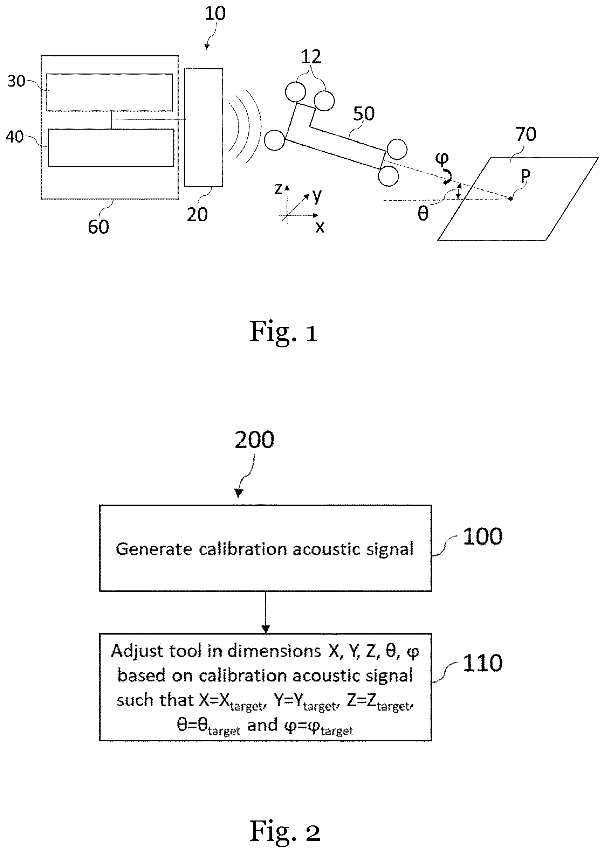

Fig. 1 shows a schematic illustration of an alignment system 10 for adjusting a tool 50 in a plurality of dimensions according to an embodiment of the third aspect of the invention. In this exemplary embodiment, the tool 50, which can for example be a surgical tool or an industrial tool, has three translational degrees of freedom, i.e. it can be moved in three mutually orthogonal directions X, Y and Z in space, and two rotational degrees of freedom, i.e. it can be rotated about two different axes by corresponding angles θ and ϕ. Notably, this is just an example and an alignment system according to the third aspect of the invention may be configured for adjusting a tool in a different plurality of dimensions, possibly comprising a different number of degrees of freedom, for example a smaller or larger number thereof.

The system 10 comprises a plurality of spatial markers 12 that are arranged on the tool 50 and a detection system 20, which can for example be a camera configured for implementing real-time optical tracking, configured for detecting a position and orientation of the spatial markers 12 in real-time, thereby registering a state of the tool 50 in all five dimensions corresponding to the five degrees of freedom thereof: X, Y, Z , θ and ϕ.

The system 10 further comprises a processing unit 60 that is functionally connected with the detection system 20 and comprises a mapping unit 30 and an adjustment unit 40. The mapping unit 30 is configured for determining a state of the tool in the dimensions X, Y, Z , θ and ϕ with respect to a target reference frame, which is defined taking into account a target position P on an external object 70 and a target orientation (with respect to the angular degrees of freedom θ and ϕ) of the tool 50 with respect to the target position P on the external object 70. The external object 70 may for example correspond to a workpiece, to a further tool, to a set up, or to the body of a patient, with respect to which the tool 50 is to be adjusted.

The mapping unit 30 hence correlates the information provided by the detection system 20 about the position and orientation of the tool 50 in space with the target position P, which has coordinates X_{target}, Y_{target}, Z_{target} in the X-, Y- and Z direction, respectively, and with the target orientation of the tool 50, which is characterised by target angles θ_{target} and ϕ_{target}. Thus, while the detection system 20 can detect the state of the tool 50 with respect to the five dimensions X, Y, Z , θ and ϕ without any specific external reference, for example on the basis of the own reference frame of the detection system 20, the mapping unit 30 determines the state of the tool 50 with respect to a target reference frame that is defined taking into account the target position P and the target orientation to be achieved by the tool 50. For this purpose, it is for example possible to use further spatial markers (not shown) arranged on the object 70 and/or arranged with respect to the object 70 with a predefined spatial relation. Additionally or alternatively, a predefined spatial relationship between the object 70 and the detection system 20 may be used, possibly without any further spatial markers.

The adjustment unit 40 is functionally connected to the mapping unit 30 and is configured for generating an acoustic calibration signal, which can be outputted to an amplifier and/or a loudspeaker (not shown) and can assist an operator of the tool for adjusting the tool with respect to the target position P and the target orientation exclusively based on auditory feedback. It is also possible that the adjustment unit 40 includes its own amplifier and/or loudspeaker for emitting sound based on the acoustic calibration signal. Thanks to the acoustic calibration signal generated by the adjustment unit 40, the tool 50 can be adjusted using the system 10 following the method 200 schematically illustrated in Fig. 2, i.e. by generating the acoustic calibration signal and by using the acoustic calibration signal to adjust the tool 50 such that, once the adjustment is completed, the state of the tool 50 in the five dimensions in which the tool 50 has degrees of freedom corresponds to the target state, i.e. such that X=X_{target}, Y=Y_{target}, Z=Z_{target}, θ=θ_{target} and ϕ=ϕ_{target}.

As shown in Fig. 2, a method 200, which corresponds to a method according to the second aspect of the invention and can be performed using the system 10 of Fig. 1 relying on the acoustic calibration signal generated by the adjustment unit 40, comprises generating the acoustic calibration signal at 100, in particular by the adjustment unit 40, and adjusting the tool 50 at 110 in each of the dimensions X, Y, Z , θ and ϕ based on the calibration signal, such that the tool eventually reaches the target state X=X_{target}, Y=Y_{target}, Z=Z_{target}, θ=θ_{target} and ϕ=ϕ_{target}. Notably, 100 and 110 can overlap in time with each other.

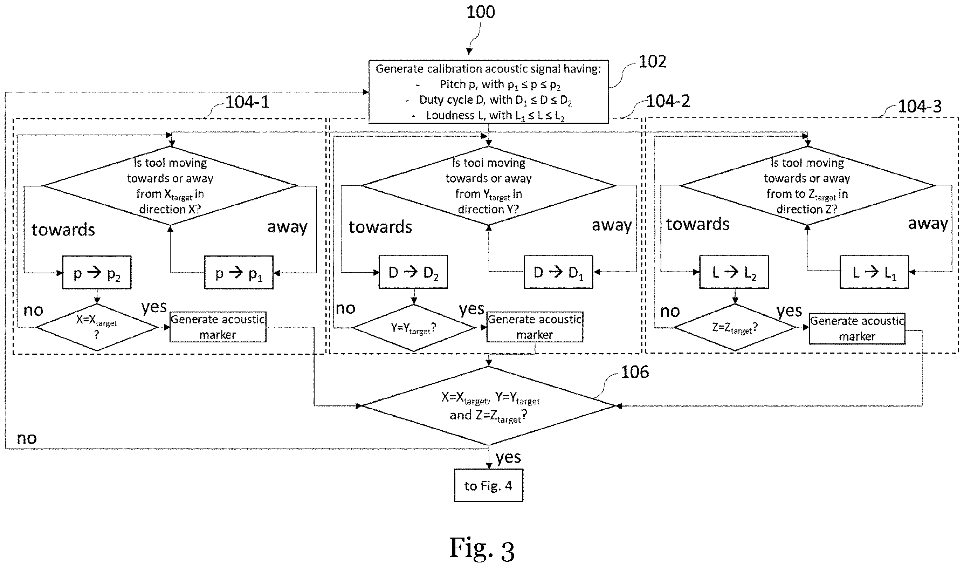

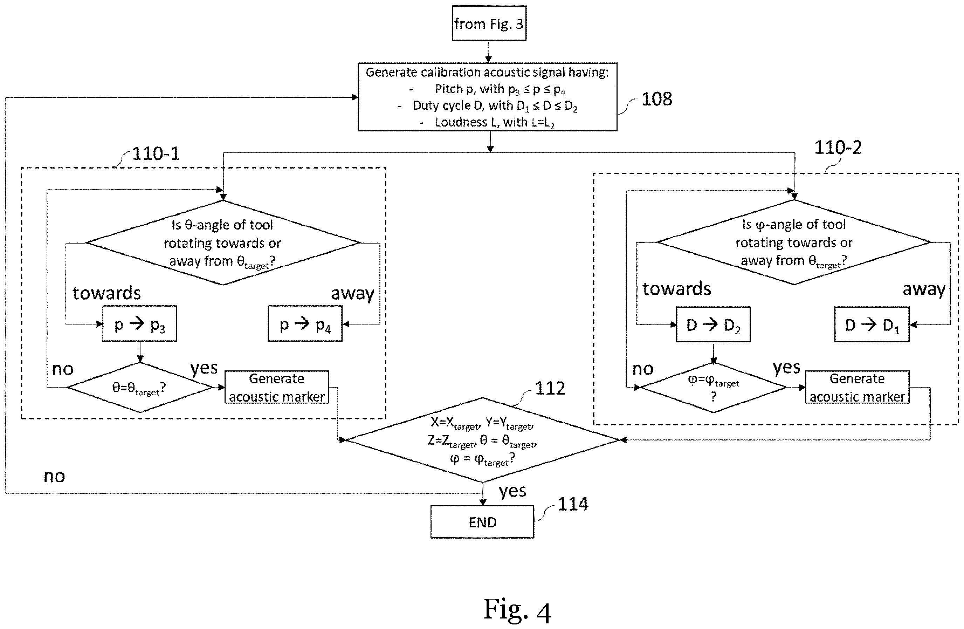

Figs. 3 and 4 show a schematic diagram of an example of a method 100 of generating the acoustic calibration signal, which corresponds to a method according to the first aspect of the invention. The method 100 can be implemented by the adjustment unit 40.

The method 100 comprises generating the acoustic calibration signal as an intermittent signal having three different acoustic properties: a pitch p, a duty cycle D and a loudness L.

The pitch p is associated to the state of the tool 50 in the X direction. The pitch p is comprised within a predetermined pitch range having a lower limit p₁ and an upper limit p₂, i.e. having p₁ ≤ p ≤ p2, for example 330 Hz ≤ p ≤ 440 Hz. As shown in functional block 104-1, the pitch p of the acoustic calibration signal varies towards the predetermined value p₂ (the upper limit) when the detection system 20 and the mapping unit 30 detect that the tool 50 is moving towards the target adjustment X=X_{target} in the X direction, whereas the pitch p varies towards the predetermined value p₁ (the lower limit) when the detection system 20 and the mapping unit 30 detect that the tool 50 is moving away from the target adjustment X=X_{target} in the X direction. When the detection system 20 and the mapping unit 30 detect that the position of the tool 50 in the X direction corresponds to the target adjustment X=X_{target} within a predefined tolerance of for example ± 1 mm, an acoustic marker is generated, for example in the form of a first arpeggio. The pitch then stays constant at p=p₂ as long as the position of the tool 50 in the X direction corresponds to the target adjustment X=X_{target} within said predefined tolerance.

The duty cycle D is associated to the state of the tool 50 in the Y direction. The duty cycle is comprised within a predetermined duty cycle range having a lower limit D₁ and an upper limit D₂, i.e. having D₁ ≤ D ≤ D₂, for example 0.2 ≤ D ≤ 1. As shown in functional block 104-2, the duty cycle D of the acoustic calibration signal varies towards the predetermined value D₂ (the upper limit) when the detection system 20 and the mapping unit 30 detect that the tool 50 is moving towards the target adjustment Y_{target} in the Y direction, whereas the duty cycle varies towards the predetermined value D₁ (the lower limit) when the detection system 20 and the mapping unit 30 detect that the tool 50 is moving away from the target adjustment Y=Y_{target} in the Y direction. When the detection system 20 and the mapping unit 30 detect that the position of the tool 50 in the Y direction corresponds to the target adjustment Y=Y_{target} within a predefined tolerance of for example ± 1 mm, an acoustic marker is generated, for example in the form of a second arpeggio, which can be different from the first arpeggio. The duty cycle then stays constant at D=D₂ as long as the position of the tool 50 in the Y direction corresponds to the target adjustment Y=Y_{target} within said predefined tolerance.

The loudness L is associated to the state of the tool 50 in the Z direction. The loudness is comprised within a predetermined loudness range having a lower limit L₁ and an upper limit L₂, i.e. having L₁ ≤ L ≤ L₂, for example 50 dB ≤ L ≤ 65 dB. As shown in functional block 104-3, the loudness L of the acoustic calibration signal varies towards the predetermined value L₂ (the upper limit) when the detection system 20 and the mapping unit 30 detect that the tool 50 is moving towards the target adjustment Z_{target} in the Z direction, whereas the loudness varies towards the predetermined value L₁ (the lower limit) when the detection system 20 and the mapping unit 30 detect that the tool 50 is moving away from the target adjustment Z=Z_{target} in the Z direction. When the detection system 20 and the mapping unit 30 detect that the position of the tool 50 in the Z direction corresponds to the target adjustment Z=Z_{target} within a predefined tolerance of for example ± 2 mm, an acoustic marker is generated, for example in the form of a third arpeggio, which can be different from the first and/or second arpeggios. The loudness then stays constant at L=L2 as long as the position of the tool 50 in the Z direction corresponds to the target adjustment Z=Z_{target} within said predefined tolerance.

The pitch, the duty cycle and the loudness of the acoustic calibration signal can be acoustically perceived by a user of the system 10 of Fig. 1 simultaneously. Thus, it is possible to rely on the acoustic calibration signal to correctly adjust the position of the tool 50 in the directions X, Y and Z to the target position P=(X_{target}, Y_{target}, Z_{target}). Blocks 104-1, 104-2 and 104-3 operate simultaneously.

Once the tool 50 is correctly adjusted in the directions X, Y and Z to the target position P=(X_{target}, Y_{target}, Z_{target}), the adjustment unit 40 monitors this adjustment in the X, Y and Z directions (cf. block 106 in Fig. 3). As long as the adjustment in directions X, Y and Z to the target position P=(X_{target}, Y_{target}, Z_{target}) is maintained, the method 100 proceeds to block 108 in Fig. 4. Otherwise, if adjustment in one of the directions X, Y or Z to the corresponding target position P=(X_{target}, Y_{target}, Z_{target}) is lost, the method 100 goes back to block 102 to correctly adjust the tool in the corresponding directions X, Y and/or Z again.

In block 108 (see Fig. 4), the acoustic calibration signal continues to be generated having a pitch p, a duty cycle D and a loudness L. However, the pitch now varies associated to the θ-angle of the tool 50 and the duty cycle D now varies associated to the ϕ-angle of the tool 50. The loudness remains constant.

The pitch p is no longer comprised within the predetermined pitch range p₁ ≤ p ≤ p₂ but within a different predetermined pitch range having a lower limit p₃ and an upper limit p₄, i.e. having p₃ ≤ p ≤ p₄, for example 550 Hz ≤ p ≤ 660 Hz. This means that, when the tool is correctly adjusted in the directions X, Y and Z to the target position P=(X_{target}, Y_{target}, Z_{target}) and starts being adjusted in the angular dimensions θ and ϕ, the pitch undergoes a sudden increase from 440 Hz to at least 550 Hz, i.e. of at least 25%. This sudden increase in the pitch of the acoustic calibration signal is a further acoustic marker that indicates to a user of the system 10 that the tool is correctly adjusted in the directions X, Y and Z to the target position P=(X_{target}, Y_{target}, Z_{target}) and is now to be adjusted with respect to the angular dimensions θ and ϕ.

As shown in functional block 110-1, the pitch p of the acoustic calibration signal varies towards the predetermined value p₄ (the upper limit) when the detection system 20 and the mapping unit 30 detect that the tool 50 is rotating towards the target adjustment θ = θ_{target}, whereas the pitch p varies towards the predetermined value p₃ (the lower limit) when the detection system 20 and the mapping unit 30 detect that the tool 50 is rotating away from the target adjustment θ = θ_{target}. When the detection system 20 and the mapping unit 30 detect that the orientation of the tool 50 corresponds to the target adjustment θ = θ_{target} within a predefined tolerance of for example ± 1°, an acoustic marker is generated, for example in the form of a low tone. The pitch then stays constant at p=p₄ as long as the orientation of the tool 50 corresponds to the target adjustment θ = θ_{target} within the predefined tolerance.

Meanwhile, as shown in functional block 110-2, the duty cycle D of the acoustic calibration signal varies towards the predetermined value D₂ (the upper limit) when the detection system 20 and the mapping unit 30 detect that the tool 50 is rotating towards the target adjustment ϕ = ϕ_{target}, whereas the duty cycle D varies towards the predetermined value D₁ (the lower limit) when the detection system 20 and the mapping unit 30 detect that the tool 50 is rotating away from the target adjustment ϕ = ϕ_{target}. When the detection system 20 and the mapping unit 30 detect that the orientation of the tool 50 corresponds to the target adjustment ϕ = ϕ_{target} within a predefined tolerance of for example ± 2°, an acoustic marker is generated, for example in the form of a low tone. The duty cycle then stays constant at D=D₂ as long as the orientation of the tool 50 corresponds to the target adjustment ϕ = ϕ_{target} within the predefined tolerance.

Thus, according to the exemplary embodiment illustrated in Figs. 3 and 4, it is possible to rely on the acoustic calibration signal to correctly adjust the position of the tool 50 first in the directions X, Y and Z to the target position P=(X_{target}, Y_{target}, Z_{target}) and then in the angular dimensions θ and ϕ to correctly orient the tool to the target orientation θ = θ_{target}, ϕ = ϕ_{target}, thereby reaching the target state X=X_{target}, Y=Y_{target}, Z=Z_{target}, θ=θ_{target} and ϕ=ϕ_{target}. It is checked at 112 that X=X_{target}, Y=Y_{target}, Z=Z_{target}, θ=θ_{target} and ϕ=ϕ_{target}, then the adjustment process ends at 114.

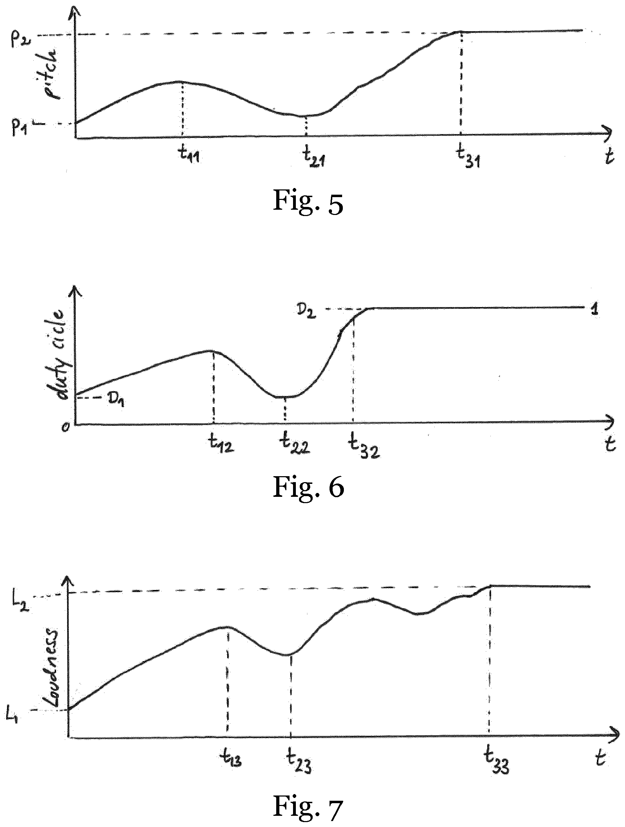

Fig. 5 shows an exemplary evolution in time of the pitch p of the acoustic calibration signal of the embodiments described above with respect to Figs. 1 to 3 (blocks 102 to 106 in Fig. 3). In this example, it is assumed that at an initial time, the pitch has a value p=p₁, e.g. p = 330 Hz, corresponding to a state in which the tool 50 is furthest away from the target position X=X_{target} in the X direction. Between the initial time and a time t₁₁, the tool 50 is moving in the X direction approaching the position X=X_{target} and this is reflected in the calibration signal by a pitch increase. Between the time t₁₁ and a time t₂₁, the tool 50 is moving in the X direction away from the position X=X_{target} and this is reflected in the calibration signal by a pitch decrease. Between the time t₂₁ and a time t₃₁, the tool 50 is moving again in the X direction continuously approaching the target position X=X_{target} and this is reflected in the calibration signal by a continuous pitch increase up to the value p=p₂. At the time t₃₁, the tool 50 reaches the target position X=X_{target} in the X direction and so the pitch of the acoustic calibration signal stays at the value p=p₂, e.g. p = 440 Hz from this time on.

Fig. 6 shows an exemplary evolution of the duty cycle D of the acoustic calibration signal of the embodiments described above with respect to Figs. 1 to 3 in time (blocks 102 to 106 in Fig. 3). The time evolution of the duty cycle illustrated in Fig. 6 can be simultaneous with the time evolution of the pitch illustrated in Fig. 5. In this example, it is assumed that at an initial time, the duty cycle has a value D=D₁, e.g. D = 0.2, corresponding to a state in which the tool 50 is furthest away from the target position Y=Y_{target} in the Y direction. Between the initial time and a time t₁₂, the tool 50 is moving in the Y direction approaching the position Y=Y_{target} and this is reflected in the calibration signal by an increase in duty cycle. Between the time t₁₂ and a time t₂₂, the tool 50 is moving in the Y direction away from the position Y=Y_{target} and this is reflected in the calibration signal by a decrease in duty cycle. Between the time t₂₂ and a time t₃₂, the tool 50 is moving again in the Y continuously approaching the target position Y=Y_{target} and this is reflected in the calibration signal by a continuous increase in duty cycle up to the value D=D₂. At the time t₃₂, the tool 50 reaches the target position Y=Y_{target} in the Y direction and so the duty cycle of the acoustic calibration signal stays at the value D=D₂, e.g. D = 1 (continuous non-intermittent signal) from this time on.

Fig. 7 shows an exemplary evolution of the loudness L of the acoustic calibration signal of the embodiments described above with respect to Figs. 1 to 3 in time (blocks 102 to 106 in Fig. 3). The time evolution of the loudness illustrated in Fig. 7 can be simultaneous with time evolution of the pitch illustrated in Fig. 5 and the time evolution of the duty cycle illustrated in Fig. 6. In this example, it is assumed that at an initial time, the loudness has a value L=L₁, e.g. L = 50 dB, corresponding to a state in which the tool 50 is furthest away from the target position Z=Z_{target} in the Z direction. Between the initial time and a time t₃₁, the tool 50 is moving in the Z direction approaching the position Z=Z_{target} and this is reflected in the calibration signal by an increase in loudness. Between the time t₁₃ and a time t₂₃, the tool 50 is moving in the Z direction away from the position Z=Z_{target} and this is reflected in the calibration signal by a decrease in loudness. Between the time t₂₃ and a time t₃₃, the tool 50 is moving again in the Z direction, towards the target position Z=Z_{target} and away from it but eventually reaching the target position Z=Z_{target} and this is reflected in the calibration signal by corresponding increases and decreases in the loudness, which maintains the value L=L₂, e.g. L= 65 dB, from the time t₃₃ on.

The time evolution of the pitch according to Fig. 5, the time evolution of the duty cycle according to Fig. 6 and the time evolution of the loudness according to Fig. 7 can be simultaneously perceived by the human ear for one and the same acoustic calibration signal. The human ear can perceive whether the pitch of the acoustic calibration signal is increasing or decreasing (or staying constant), and can further simultaneously and independently perceive whether the duty cycle and the loudness is increasing or decreasing (or staying constant).

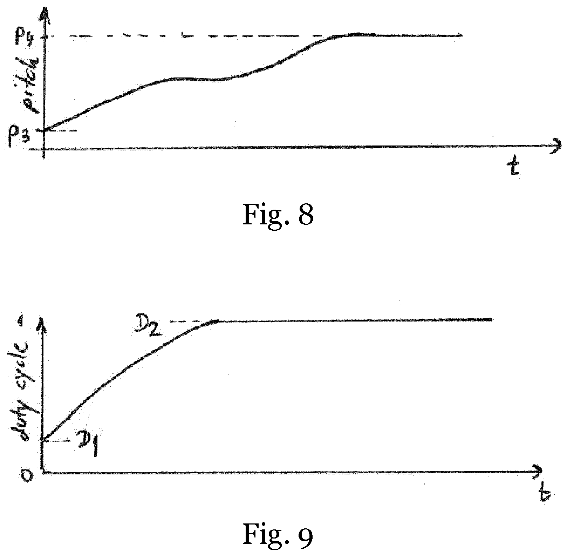

Figs. 8 and 9 respectively show an exemplary evolution of the pitch and a duty cycle of the acoustic calibration signal the embodiments described above with respect to Figs. 1, 2 and 4 in time (cf. blocks 108 to 112 in Fig. 4). In contrast to the situation in Fig. 5, when the tool 50 was being adjusted in the X direction, the pitch p of the acoustic calibration signal is no longer varying within the predetermined range p₁ ≤ p ≤ p₂ but instead within a predetermined range p₃ ≤ p ≤ p₄, with p₃ being for example 550 Hz and p₄ being for example 660 Hz and is now associated to the θ-angle of the tool 50. As shown in Fig. 8, the tool continuously rotates towards the target angle θ=θ_{target} and this is reflected by a corresponding continuous increase of the pitch from a value p=p₃ to a value p=p₄. Meanwhile, as shown in Fig. 9, the duty cycle D, which is now associated to the ϕ-angle of the tool 50, continuously increases from the initial value D=D₁ to the value D=D₂ signaling a corresponding continuous approach of the orientation of the tool 50 to the target angle ϕ=ϕ_{target}.

The time evolution of the pitch according to Fig. 8, the time evolution of the duty cycle according to Fig. 9 can be simultaneously perceived by the human ear for one and the same acoustic calibration signal.

With reference to Figs. 3 and 4, Figs. 5, 6 and 7 may represent the acoustic calibration signal between blocks 102 and 106, while Figs. 8, 9 and 7 may represent the acoustic calibration signal between blocks 108 and 112.

Notably, in other embodiments of the invention, a tool can be adjusted in two dimensions, each of the dimensions being associated to pitch but evolving in different pitch ranges. For example, the tool could be adjusted in an X-direction associated to a pitch evolving in the range p₁ ≤ p ≤ p₂ as exemplary shown in Fig. 5 while simultaneously being adjusted in a Y-direction associated to a pitch evolving the in the range p₃ ≤ p ≤ p₄, as exemplary shown in Fig. 8. The final value p₄ of the pitch range for the Y-direction can be chosen to be consonant with the final value p₂ of the pitch range for the X-direction, for example an octave, a perfect fourth or a perfect fifth thereof, e.g. p₂=440Hz and p₄=660 Hz. This allows a user adjusting the tool in the X and Y dimensions to identify when the tool has been correctly adjusted in both dimensions by identifying a consonant tone.

Although preferred exemplary embodiments are shown and specified in detail in the drawings and the preceding specification, these should be viewed as purely exemplary and not as limiting the invention. It is noted in this regard that only the preferred exemplary embodiments are shown and specified, and all variations and modifications should be protected that presently or in the future lie within the scope of protection of the invention as defined in the claims.

## Claims

1. A method for generating an acoustic calibration signal for adjusting a tool in a plurality of dimensions, each dimension of the plurality of dimensions corresponding to a respective degree of freedom of the tool, wherein the method comprises:
generating an acoustic calibration signal with a plurality of acoustic properties comprising an acoustic property for each dimension of the plurality of dimensions,
wherein each acoustic property of the plurality of acoustic properties varies towards a corresponding predetermined value when the tool is being adjusted in the corresponding dimension towards a corresponding predetermined target adjustment.

2. The method of claim 1, wherein each acoustic property of the plurality of acoustic properties varies away from the corresponding predetermined value when the tool is being adjusted in the corresponding dimension away from the corresponding predetermined target adjustment.

3. The method of claim 1 or 2, wherein at least one acoustic property of the plurality of acoustic properties corresponds to one of pitch, pulsing frequency, duty cycle, loudness, and tone colour of the acoustic calibration signal, wherein preferably at least another acoustic property of the plurality of acoustic properties corresponds to one of pitch, pulsing frequency, duty cycle, loudness and tone colour of the acoustic calibration signal.

4. The method of any of the preceding claims, wherein at least one dimension of the plurality of dimensions corresponds to a translational degree of freedom, a rotational degree of freedom or an orientational degree of freedom of the tool, and/or wherein at least another dimension of the plurality of dimensions corresponds to another translational degree of freedom, rotational degree of freedom or orientational degree of freedom of the tool.

5. The method of any of the preceding claims, wherein at least one acoustic property of the plurality of acoustic properties is kept constant, optionally at the corresponding predetermined value, while the tool remains adjusted in the corresponding dimension at the corresponding predetermined target adjustment.

6. The method of any of the preceding claims, wherein the method further comprises, for at least one dimension of the plurality of dimensions, generating an acoustic marker when the tool reaches the corresponding predetermined target adjustment in said at least one dimension, in particular within a predefined tolerance;
wherein the at least one generated acoustic marker optionally corresponds to a discontinuous variation of the corresponding acoustic property, in particular by 5% or more, preferably by 10% or more, more preferably by 20% or more; and/or
wherein generating the at least one acoustic marker preferably comprises generating a corresponding additional acoustic signal different from the acoustic calibration signal.

7. The method of any of the preceding claims, wherein at least one acoustic property of the plurality of acoustic properties varies within a corresponding predetermined range towards one end value of said corresponding predetermined range when the tool is being adjusted in the respective dimension towards the corresponding predetermined target adjustment and preferably towards another end value of said corresponding predetermined range when the tool is being adjusted in the respective dimension away from the corresponding predetermined target adjustment.

8. The method of claims 6 and 7, wherein generating the at least one acoustic marker comprises replacing the corresponding predetermined range by a new corresponding predetermined range different from said corresponding predetermined range.

9. The method of any of the preceding claims, wherein at least a first acoustic property and a second acoustic property of the plurality of acoustic properties correspond to pitch,
wherein the first acoustic property varies within a first predetermined range towards an end value of the first predetermined range when the tool is being adjusted in the first dimension towards a first predetermined target adjustment, and
wherein the second acoustic property varies within a second predetermined range, in particular different from the first predetermined range, towards one end value of the second predetermined range when the tool is being adjusted in the second dimension towards a second predetermined target adjustment;
wherein said end value of the second predetermined range preferably is an octave, a perfect fifth or a perfect fourth of said end value of the first predetermined range.

10. The method of any of the preceding claims, wherein the plurality of dimensions comprises a first dimension corresponding to a first degree of freedom of the tool and a second dimension corresponding to a second degree of freedom of the tool,
wherein the plurality of acoustic properties comprises a first acoustic property and a second acoustic property different from the first acoustic property, wherein the first and second acoustic properties are in particular simultaneously perceivable acoustic properties;
wherein the plurality of dimensions optionally further comprises a third dimension corresponding to a third degree of freedom of the tool,
wherein the plurality of acoustic properties further optionally comprises a third acoustic property different from the first and second acoustic properties, wherein the first, second and third acoustic properties are in particular simultaneously perceivable acoustic properties.

11. The method of claim 10, wherein the plurality of dimensions further comprises a third dimension corresponding to a third degree of freedom of the tool and a fourth dimension corresponding to a fourth degree of freedom of the tool,
wherein the first acoustic property varies within a first predetermined range towards one end value of the first predetermined range when the tool is being adjusted in the first dimension towards a first predetermined target adjustment and preferably towards another end value of the first predetermined range when the tool is being adjusted in the first dimension away from the first predetermined target adjustment, and/or
wherein the second acoustic property varies within a second predetermined range towards one end value of the second predetermined range when the tool is being adjusted in the second dimension towards the second predetermined target adjustment and preferably towards another end value of the second predetermined range when the tool is being adjusted in the second dimension away from the second predetermined target adjustment; and
wherein, when the tool reaches the first predetermined target adjustment in the first dimension, in particular within a predefined tolerance, and/or reaches the second predetermined target adjustment in the second dimension, in particular within a predefined tolerance:
the first acoustic property varies within a third predetermined range different from the first predetermined range towards one end value of the third predetermined range when the tool is being adjusted in the third dimension towards a third predetermined target adjustment and preferably towards another end value of the third predetermined range when the tool is being adjusted in the third dimension away from the third predetermined target adjustment, and/or
the second acoustic property varies within a fourth predetermined range different from the second predetermined range towards one end value of the fourth predetermined range when the tool is being adjusted in the fourth dimension towards the a fourth predetermined target adjustment and preferably towards another end value of the fourth predetermined range when the tool is being adjusted in the fourth dimension away from the fourth predetermined target adjustment;
wherein optionally, when the tool deviates from the first predetermined target adjustment in the first dimension, in particular beyond a predefined tolerance, the first acoustic property stops varying within the third predetermined range and retakes varying within the first predetermined range towards said one end value of the first predetermined range when the tool is being adjusted in the first dimension towards the first predetermined target adjustment and preferably towards said another end value of the first predetermined range when the tool is being adjusted in the first dimension away from the first predetermined target adjustment; and/or
wherein optionally, when the tool deviates from the second predetermined target adjustment in the second dimension, in particular beyond a predefined tolerance, the second acoustic property stops varying within the fourth predetermined range and retakes varying within the second predetermined range towards said one end value of the second predetermined range when the tool is being adjusted in the second dimension towards the second predetermined target adjustment and preferably towards said another end value of the second predetermined range when the tool is being adjusted in the second dimension away from the second predetermined target adjustment.

12. A method for adjusting a tool in a plurality of dimensions, each dimension corresponding to a degree of freedom of the tool, wherein the method comprises:
generating an acoustic calibration signal according to the method of any of the preceding claims; and
adjusting the tool in each dimension of the plurality of dimensions based on the generated acoustic calibration signal, such that for a plurality of acoustic properties of the generated acoustic calibration signal, each acoustic property varies towards a corresponding predetermined, in particular until each acoustic property reaches the corresponding predetermined value.

13. An alignment system (10) for adjusting a tool (50) in a plurality of dimensions, each dimension corresponding to a degree of freedom of the tool (50), wherein the system (10) comprises:
a plurality of spatial markers (12) arrangeable on the tool (50) for marking a state of the tool (50) in each dimension of the plurality of dimensions;
a detection system (20) configured for registering the state of the tool (50) in each dimension of the plurality of dimensions marked by the spatial markers (12);
a mapping unit (30) for determining a target state of the tool (50) in each dimension of the plurality of dimensions with respect to a target reference frame; and
an adjustment unit (40) configured for generating an acoustic calibration signal according to the method of any of claims 1 to 11, wherein the acoustic calibration signal is generated with a plurality of acoustic properties, wherein each acoustic property of the plurality of acoustic properties varies towards a corresponding predetermined value when the tool (50) is being adjusted in the corresponding dimension towards a corresponding predetermined target adjustment,
wherein the target state determined by the mapping unit (30) corresponds to a state in which the tool (50) is adjusted in each dimension of the plurality of dimensions to the corresponding predetermined target adjustment, in particular within a predefined tolerance.

14. The alignment system (10) of claim 13, wherein the adjustment unit (40) is further configured for generating the acoustic calibration signal such that each acoustic property varies away from said corresponding predetermined value when the tool (50) is being adjusted in said corresponding dimension away from said predetermined target adjustment.

15. The alignment system (10) of claim 13 or 14, wherein the adjustment unit (40) is further configured for generating, for at least one dimension of the plurality of dimensions, an acoustic marker when the tool (50) reaches the corresponding predetermined target adjustment in said at least one dimension, in particular within the predefined tolerance.
